# EUROPEAN PATENT SPECIFICATION

(11) **EP 2 753 392 B1**
(45) Date of publication and mention of the grant of the patent: **02.01.2019**
(21) Application number: 12830568.7
(22) Date of filing: 07.09.2012
(51) Int. Cl.: A61M 16/06

(54) **OXYGEN FACEMASK WITH CAPNOGRAPHY MONITORING PORTS**
SAUERSTOFFGESICHTSMASKE MIT KAPNOGRAFIEÜBERWACHUNGS-PORTS
MASQUE FACIAL À OXYGÈNE AVEC ORIFICES DE SURVEILLANCE DE CAPNOGRAPHIE

(30) Priority: 07.09.2011 US 201113227345
(43) Date of publication of application: 16.07.2014
(73) Proprietor: Monitor Mask Inc., Seattle, WA 98105 (US)
(72) Inventor: BEARD, John, W., Lafayette, CA 94549 (US)
(74) Representative: Sadler, Peter Frederick
(86) International application number: PCT/US2012/054268
(87) International publication number: WO 2013/036839

(56) References cited:
- EP-A2- 1 027 905
- US-A- 4 201 205
- US-A- 5 400 781
- US-A- 5 474 060
- US-A- 5 586 551
- US-A1- 2006 196 510
- US-A1- 2007 023 040
- US-A1- 2011 203 591
- A. R. EITKENKHEDA ET AL.: 'Meditsina' RUKOVODSTVO PO ANESTEZIOLOGII. POD RED. 1999, MOSKVA, page 456, XP008173255
- V. L. KASSIL ET AL.: 'Meditsina' RASPIRATORNAYA PODDERZHKA. RUKOVODSTVO DLYA VRACHEI. 1997, MOSKVA, page 290, XP008173196

## Description

### FIELD

The present invention relates to oxygen delivering face masks.

### BACKGROUND

A steady inflow of oxygen is required to sustain human life. A short interruption or reduction in a person's oxygen supply can rapidly lead to brain or body damage, or death. An individual with too little oxygen in his blood (hypoxemia) or at risk for developing hypoxemia may be given oxygen. An individual able to breathe on his own may be given supplemental oxygen therapy for various reasons and in various places. Oxygen may be given to an individual who has shortness of breath or COPD (chronic obstructive pulmonary disease). Supplemental oxygen maybe delivered to a patient who has suffered trauma or an acute myocardial infarction (heart attack). Supplemental oxygen may be given during certain surgical interventions or during post-anesthesia recovery after a surgical intervention. Supplemental oxygen may be given anywhere. It may be given, for example, in a person's home, in a clinic or in a hospital such as in a trauma center, an emergency room, an operating room, a recovery room, or an intensive care unit. A person who is receiving supplemental oxygen therapy is generally weak, injured, or compromised in some way. Such a person is prone to stop breathing briefly or altogether. In order to determine if a person receiving supplemental oxygen is continuing to breathe, an assay may be performed. A non-invasive, expiratory gas sampling device may be used to determine if the person is exhaling as evidence he is continuing to breathe. Commonly, the expiratory gas sampled is carbon dioxide.

Both face masks and nasal cannula have been used to deliver supplemental oxygen and to sample carbon dioxide. U.S. 5,400,781 to Davenport discloses an oxygen mask with two openings in the floor of the chamber in front of the mouth that lead to an oxygen source and a carbon dioxide monitor. U.S. 5,474,060 to Evans describes an oxygen mask with an inlet for directing a flow of gas (oxygen) to the interior of the mask, and a port for allowing the exhaled air to flow through and a tube for directing the exhaled air to a monitoring apparatus. U.S. 6,247,470 to Ketchedjian uses a flexible lever arm near the face and connected to tubing to deliver oxygen and sample exhaled gases. U.S. 6,439,234 to Curti describes a nasal cannula with two prongs, with the first prong for delivering oxygen and the second prong for sampling carbon dioxide. WO 91/14469 teaches a nasal gas cannula and an oral gas capture member for delivering and capturing carbon dioxide. Further cited published patent applications describe a variety of face masks for delivering oxygen to a patient. These include US 2011/203591, EP 1 027 905, US 2006/196510, US 5 586 551, US 4 201 205.

Although these face masks and cannulas attempt to solve some of the problems with delivering oxygen to an individual and determining if he is breathing, none provides an easy to use, universal device that can deliver oxygen and sample an expiratory gas in a variety of circumstances. The present invention is directed to meeting these, as well as other, needs.

### SUMMARY OF THE DISCLOSURE

Described herein are devices, methods, systems, and kits useful for administering oxygen and/or sampling gases from a mammalian body. The devices are particularly useful for sampling carbon dioxide, though they may be used as a part of any appropriate treatment procedure.

The present invention provides a face mask as defined in the appended claim 1. In some embodiments, the face mask includes an oxygen inlet port having a center, and at least one of the sampling ports is at least about 20 mm away from the center of the oxygen inlet port.
In some embodiments, the first and second sampling ports are at a distance of 1mm to 15 mm from the respective first and second vents.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 is a front view of an oxygen face mask with lateral sampling ports according to one aspect of the disclosure.
FIG. 2 is a side view of a face mask with a lateral monitoring port according to one embodiment.
FIG. 3 is a side view of a face mask such as the one shown in FIGS. 1 and 2 in use on a patient.
FIG. 4 shows a front view of a face mask with sampling conduit connected to one of the lateral sampling ports.
FIG. 5A shows a short face mask to allow access to an individual's mouth and face.
FIG. 5B shows a bottom portion of a face mask removed from a full mask to form the short mask shown in FIG. 5A, according to one aspect of the disclosure.
FIG. 6 shows a face mask kit according to one aspect of the disclosure.
FIG. 7 shows a side view of a face mask with vents arranged around a sampling port
FIG. 8 shows a front view of a face mask with a one-way valve covering a series of vents.
FIG. 9 shows a front view of another face mask with an opening in the front.
FIG. 10 shows a side view of a face mask such as the one shown in FIG. 9.
FIGS. 11A-B show a swivel mechanism for a gas conduit for use on a face mask.
FIG. 12 shows a face mask such as the one shown in FIG. 10 on a user.
FIG. 13A shows an embodiment of a face mask with a connector for moving a gas conduit.
FIGS. 13 B-C show a connector such as the one shown in FIG. 13A. FIG. 13D shows the degrees of freedom that a connector may have.
FIGS. 14A-C shows other embodiments of face masks and connectors for moving gas conduits.
FIG. 15 shows a front view of a face mask with a connector for moving a gas conduit and a single sampling port.
FIG. 16 shows another embodiment of a face mask with a removable cap covering each lateral sampling port.
FIGS. 17A-F show face masks, systems, and methods for setting up and using a face mask and an anesthesia breathing circuit.

### DETAILED DESCRIPTION

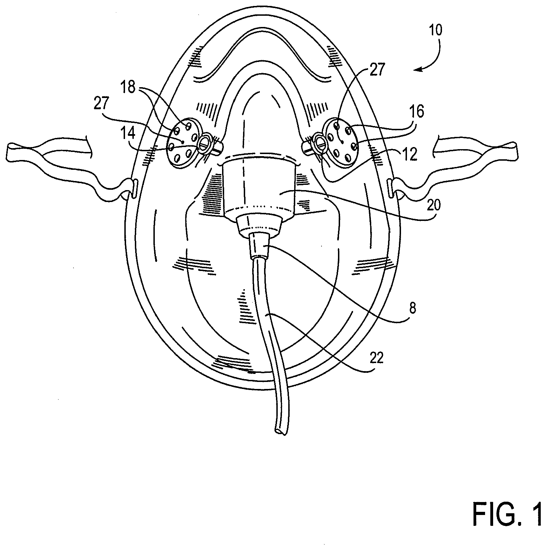

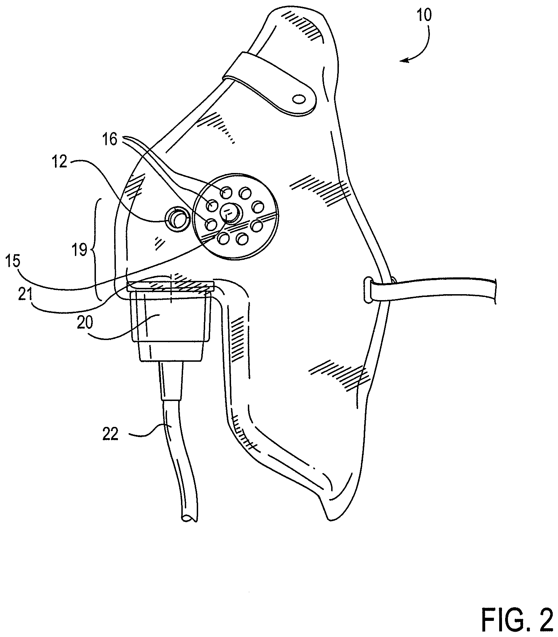

The present invention includes a universal oxygen face mask for delivering oxygen and sampling a respiratory gas for use in a variety of clinical scenarios for an individual able to breathe on his own, but requiring some supplemental oxygen. Respiratory gas (e.g. carbon dioxide) may be monitored using the face mask to ensure that the individual continues to breathe. Ensuring that the individual is breathing may be especially important when an individual is under sedation or has recently experienced a status change such as a surgical procedure or trauma. The face mask may have two (or more) lateral sampling ports for sampling a respiratory gas. The ports may be located between a level of the nose and a level of the mouth when the face mask is in use. FIG. 1 (front view) and FIG. 2 (side view) show face mask 10 embodying features of the invention including left lateral sampling port 12 and right lateral sampling port 14. Having two lateral sampling ports makes the face mask easier to use and allows for better samples to be taken. This may be the case even though, in practice, a sample may be taken from only one of the ports. A second (or additional ports beyond the second) lateral port may be unused. A face mask with two lateral sampling ports allows the mask to be used in nearly all clinical scenarios; face mask manufacturing can be streamlined and the best mask for almost any situation is readily available. A face mask with at least two lateral sampling ports eliminates the need to have a series of different masks for different purposes or reduces the number of different types of masks that may be needed. A face mask having two lateral sampling ports may be the standard for use with most or all patients, and the use of interchangeable components coupled with the face mask for specific clinical scenarios may be used in the care path.

A face mask according to the invention may be useful for a variety of clinical purposes in a variety of settings. A face mask may be used while a person needing oxygen is supine, lateral or prone; while a person's face is covered with a drape; during nebulizer therapy; during use of a non-rebreather mask; during use of an oxygen calibration device (e.g. a Venturi device); and/or during high flow oxygen therapy. In addition, a face mask could be used for administering oxygen and monitoring an aspect(s) of respiratory physiology, such as end tidal CO₂ and respiratory rate during a test of athletic endurance or cardiovascular health.

Ports high on the mask and lateral to the midline of the mask are more accessible. The lateral ports are easy to access in order to attach a sampling conduit (e.g. tubing) in a variety of patient positions and patient-caregiver physical arrangements. If a monitoring port is low on the mask, it may be difficult to gain access to the port. First, accessing a port if the port is in the immediate proximity to the oxygen port is challenging and there is limited space available to manipulate (e.g. attach and detach) a conduit. This could be of particular importance in a small pediatric mask. Secondly, a port located near the oxygen inflow is out of view and cumbersome to reach in the most common operating room scenario, for which the anesthesia provider is positioned at the head of the patient's bed. Any difficulty in accessing the monitoring port is magnified in challenging clinical situations such as an obese, prone, or laterally positioned patient. Additionally, the patient's neck, chin or other body part may get in the way of monitoring port access, especially in the case in which a patient is lying on his side.

The mask design of the disclosure may also achieve the aims of separating the port from the other equipment and from other lines (e.g. an oxygen input port, oxygen conduit, or oxygen bag). This separation prevents or reduces problems with the sampling port interfering with other equipment and lines as well as reducing or preventing problems with other equipment and lines interfering with sampling port access and sampling conduit access. This design also reduces or avoids unnecessary stimulation of the patient by keeping lines and monitors away from the eyes and other sensitive parts of the face. FIGS. 1 and 2 depict left, right lateral sampling ports 12, 14 positioned away from an oxygen inlet port center 21 of oxygen inlet port 20 on face mask 10, and out of the way of oxygen conduit coupler 8, shown coupling oxygen inlet port 20 with oxygen conduit 22. In one example, a center of the lateral sampling port is at least about 20 mm away from oxygen inlet port center 21 of oxygen inlet port 20.

Having two sampling ports available may allow a care provider (e.g. a physician, nurse, or other person) to choose a convenient sampling port. For example, when a patient is lying supine while undergoing a surgical procedure, the care provider performing the respiratory gas monitoring often sits at the patient's head. It is easier for the care provider to access one of the lateral ports and connect a tube or conduit to it for monitoring respiratory gas than it is to access a port that is obscured by the patient's neck and may be underneath the oxygen inlet port/oxygen conduit. Depending on various factors, one specific lateral sampling port may be a better choice for the care provider to use. Ease of attachment may be based on the positions of the care provider and/or the monitoring equipment to the patient. For example, a lateral side port can be chosen and easily and directly accessed based on ergonomic considerations such as patient position, monitor position, and caregiver position and handedness. In some circumstances, a care provider may not need to reach across the patient's face. As a patient may be conscious during a procedure when wearing an oxygen face mask, this is important. Having a hand close to the eyes may create or worsen a feeling of confinement or claustrophobia in a patient, which are common complaints from oxygen mask users.

Having at least two ports on the mask also means that if one of the ports cannot be used, a second monitoring port is still available. This may be the case, for example, when an individual is lying on one side, such as when a surgical procedure is being performed on the other side, and one of the ports is blocked.

In another example, a mask may be used (e.g. to deliver oxygen) without using a sampling port(s) to obtain a sample. In another example, samples could be removed from two (or more than two) sampling ports.

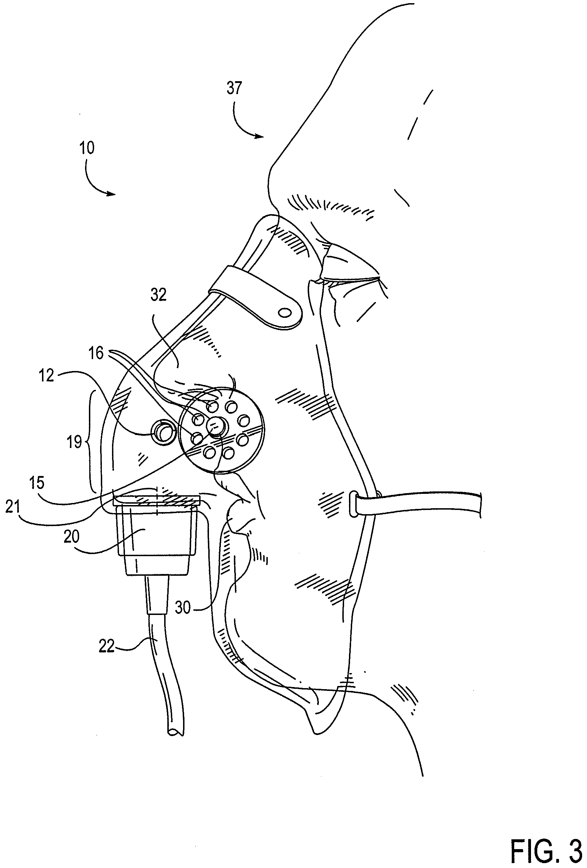

Ports may be located laterally to the midline of the mask (e.g. on opposing sides of the midline). Ports may be asymmetrically or symmetrically located relative to each other and the midline. The ports may be between a level of the nose and a level of the mouth when the mask is in use. In one example, the ports are at or below the bottom of the nose (e.g. below about a level of the nares) when the mask is in place on a mask user. In another example, the sampling ports are above the level of the lower lip. In another example, the sampling ports are above the level of the upper lip. A sampling port(s) may be positioned in any lateral position relative to the nose and mouth. A port(s) may collect nasal gases, oral gases, or both. The ports may instead or additionally collect other gases (e.g. supplemental oxygen, room air). FIG. 3 shows a side view of patient 37 wearing face mask 10 as described herein. Left lateral sampling port 12 is at a level between mouth 30 and nose 32.

A face mask may have one or more exhalation vents (e.g. exhalation ports). The face mask according to the present invention has two exhalation vents. FIGS. 1-3 show left, right exhalation vents 16, 18. An exhalation vent(s) may release or vent gas and other substance(s) from inside to outside the mask. A gas may be an expiratory gas (e.g. carbon dioxide or oxygen). Although called an exhalation vent(s), a vent may additionally allow room air or other materials to move from outside the mask to inside the mask in some embodiments. A vent(s) may move air within the mask and in particular may move air within a reservoir of the mask. A mask may have a vent(s) on a midline of the mask, or on one or both sides of the midline. There may be a plurality of exhalation vents. There may be one, two, or more exhalation vents. In one example there may be 10 or more vents. A lateral sampling port may be located outside an area encompassed by the exhalation vents, as shown in FIGS. 1-3. A lateral sampling port may be located near an exhalation vent. A lateral sampling port may be located as close to one or more exhalation vents as possible, such as left sampling port 12 located near exhalation vents 16 as shown in FIG. 3. In one example, a lateral sampling port may be located about 1 mm away from an exhalation vent. In one example, the distance between a center of a lateral sampling port and a vent is about 15 mm. In another example, a distance between a center of a lateral sampling port and a center of the vents is about 15 mm.

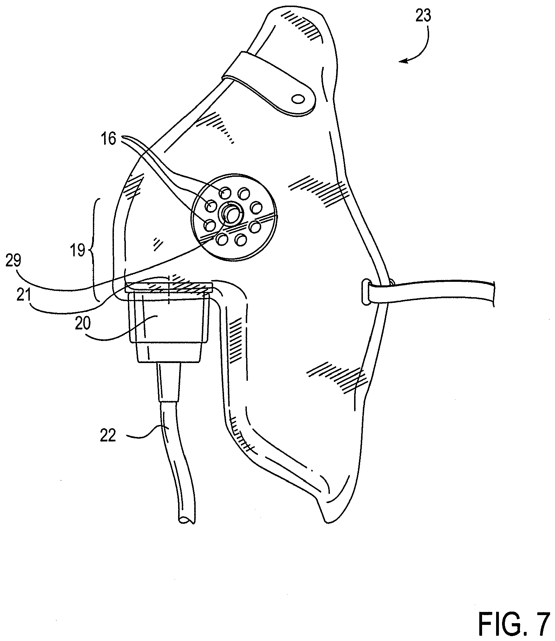

A plurality of exhalation vents (e.g. perforations) may be arranged around a lateral sampling port. A vent may define a vent center or a plurality of vents may define a vent center 27, as shown on face mask 10 in FIG.1. A port may be located at or near a vent center, substantially surrounded by exhalation vents. FIG. 7 shows face mask 23 with port 29 surrounded by a plurality of vents 16. In another example, a sampling port is outside an area of the vents and a distance between a center of a sampling port and a center of the vents is about 15 mm.

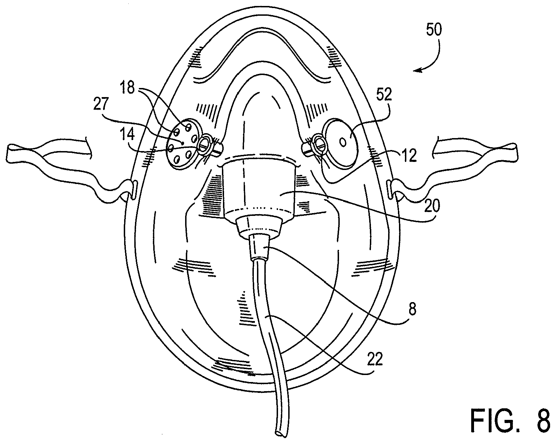

An exhalation vent may have a point of attachment (e.g. a coupling point) 15 near or at a vent center as shown in FIGS. 2 and 3. A flexible diaphragm may be coupled with a point of attachment to create a one way valve (e.g. over the vent(s)). FIG. 8 shows face mask 50 with flexible diaphragm 52 placed over the exhalation vents, creating a face mask with a one way valve. A one way valve may, for example, be used with a non-rebreather apparatus. A one way valve may allow gas inside the mask to move to outside the mask, while substantially not allowing gas outside the mask (e.g. room air) to move inside the mask. Any type of one way valve that allows flow in one direction may be used. For example, a one way valve may be single piece that is placed on or is a part of a face mask.

An exhalation vent may have low resistance to air flow as air flows out of an exhalation hole; locating a lateral sampling port near an exhalation vent may allow more accurate sampling of exhaled gas as the gas is moved past the lateral sampling port. If gas is sampled near an inflow stream of oxygen, the sampling accuracy may be lowered. This may especially be the case in high minute ventilation scenarios in which carbon dioxide levels are low and/or oxygen flow rates are high.

Mask 10 may have reservoir 19 containing a pocket of gas (e.g. air) as shown in FIGS. 2 and 3. A reservoir may allow gas mixing and provide a space near nose 32 (e.g. near the nostrils) and mouth 30 to facilitate breathing. In one example, a reservoir may extend from a level near the mouth to a level near the nose when the mask is positioned on a user. In another example, a reservoir may extend to about the bottom of the nose when the mask is in use. In one example, the reservoir extends about 50 mm vertically, 50 mm horizontally, and 50 mm in the anterior posterior dimension. A sampling port may be located in a reservoir region of the mask. As shown in FIG. 3, left lateral sampling port 12 exits the mask from reservoir 19.

Positioning a sampling port(s) away from an oxygen inlet port may make it easier (or even possible) for a care provider to change an oxygen conduit (e.g. tubing) leading to an oxygen inlet port or another connector which might not be possible (or might be very difficult) if a sampling port (or conduit connected with a sampling port) is too close to the oxygen delivery port. For example, it may be easier to change a nebulizer device coupled with the oxygen inlet port without having a sampling port nearby that might obstruct access. A sampling port may be positioned far enough away from an oxygen line connector to enable a care provider to attach both a sampling conduit and a specialized apparatus to the mask including a nebulizer, a nonrebreather, an oxygen calibration device (e.g. a Venturi device), and/or a high flow oxygen source.

A sampling port may have any shape or configuration that allows gas to move through and to connect with a conduit or sampling device. A port may be low profile and/or may be hardly visible. A sampling port may be e.g. circular, square, hexagonal, or slotted. A sampling port may have a mating part or fitting configured to removably connect with a different mating part or fitting on a conduit, including a sensing conduit. A mating part may be any as known in the art (e.g. threads, slots, pins, lock-and-key mechanism, etc.). In one example, a mating part on the sampling port is a Luer-lock that can couple with a Luer-lock on a sampling port conduit.

Any type of sampling conduit may be used. In one example, sampling conduit is a flexible polyurethane tubing. Sampling conduit may have a narrow diameter; the diameter may be smaller than a diameter of an oxygen conduit. In one example a sampling conduit may have about a 1/4 inch inner diameter or 3/8 inch outer diameter.

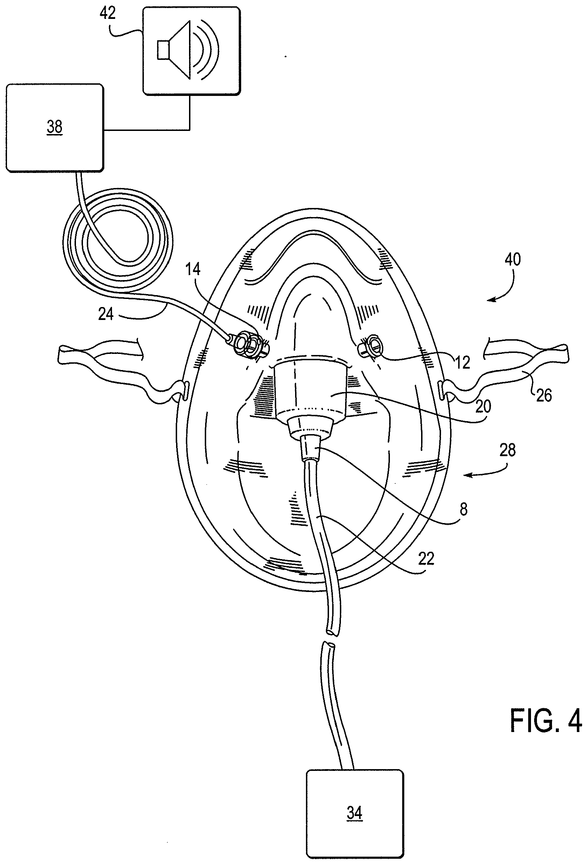

One method of using an oxygen face mask having two lateral sampling ports according to the current disclosure includes choosing a lateral sampling port and coupling a conduit with the lateral sampling port. FIG. 4 shows mask 28 with left, right lateral sampling ports 12, 14. Sampling conduit 24 is connected with right lateral sampling port 14 to enable gas (e.g. carbon dioxide) sampling according to a method of the disclosure. The method may further include the step of obtaining a sample from the port. In one example obtaining the sample comprises obtaining a sample without an anesthetic in it (e.g. without an inhaled anesthetic). The method may include the step of coupling an expiratory gas sensor to the conduit; and the expiratory gas sensor may be configured to detect carbon dioxide. The method may include the step of analyzing the sample for a component. The method may include the step of analyzing carbon dioxide (e.g. a partial pressure of carbon dioxide). The method may include the steps of removing the sampling conduit, and reattaching the conduit. The method may include the steps of providing oxygen, venting an expiratory gas, and/or administering a nebulizer or aerosol agent or treatment.

In some aspects, the method includes providing at least about 21-100% oxygen. The range includes providing room air (e.g. about 21% oxygen) to providing pure oxygen (e.g. around 100% oxygen), such as deliverable by a nonrebreather or high flow device. In some aspects, at least about 30%, at least about 40%, at least about 50%, at least about 60% oxygen, at least about 80%, at least about 90%, or more than 90% oxygen is provided. FIG 4 depicts oxygen source 34 providing oxygen through oxygen conduit 22 to oxygen inlet port 20. An oxygen source can be any as known in the art (e.g. an oxygen tank or a bag connected to an oxygen tank). In one example, monitoring may be performed without providing supplemental oxygen (e.g. only providing room air).

Any material may be sampled from the port. Any characteristic of the material may be analyzed. Gas may be sampled from the port or a component present with the gas may be sampled. A sampled gas may contain other component(s) such a therapeutic nebulized or aerosolized component or agent. A gas may be expired gas. An expired gas may be mixed, in part, with delivered oxygen, and/or room air before sampling. In one example, a gas may not contain expired air (e.g. if the patient is not breathing). In one example, carbon dioxide is sampled (capnography). In another example, oxygen is sampled. In another example, end tidal partial pressure of the gas (e.g. carbon dioxide) may be measured (or otherwise determined or calculated).

Any device or means (e.g. sensor) may be used to sample a gas. FIG. 4 shows sensor 38 coupled with sampling conduit 24 for analyzing a sample from right lateral sampling port 14. A sensor may be connected to a sampling conduit, or the conduit may be or include the sensor. Any characteristic of a gas may be sensed. An amount of a gas, a change in a level of a gas, and/or a change in a pressure of a gas may be sensed. A partial pressure of a gas may be assayed. In one example, carbon dioxide is measured and an infrared sensor is used (capnograph). In another case, carbon dioxide may be measured and a colorimetric sensor may be used (see e.g. U.S. 5,857,460 to Popitz).

A system according to the disclosure may include a face mask and one or more components that can be used with the face mask. The system may include a component configured to obtain, move, provide, sense, assay and/or measure a level of a gas. FIG. 4 shows system 40 with mask 28, sampling conduit 24, sensor 38, oxygen conduit 22, and oxygen source 34. The system may include a mask, a mask sealing agent, a face contact agent (e.g. a lotion), sampling conduit, oxygen conduit, an oxygen reservoir (e.g. partial or full rebreather reservoir), a one way valve or valve cover and/or an oxygen source (e.g. tank). In one particular example, the system includes a face mask and a sensor configured to detect a characteristic of a gas, such as a carbon dioxide partial pressure. A sensor may be coupled with or configured to be coupled with a lateral port.

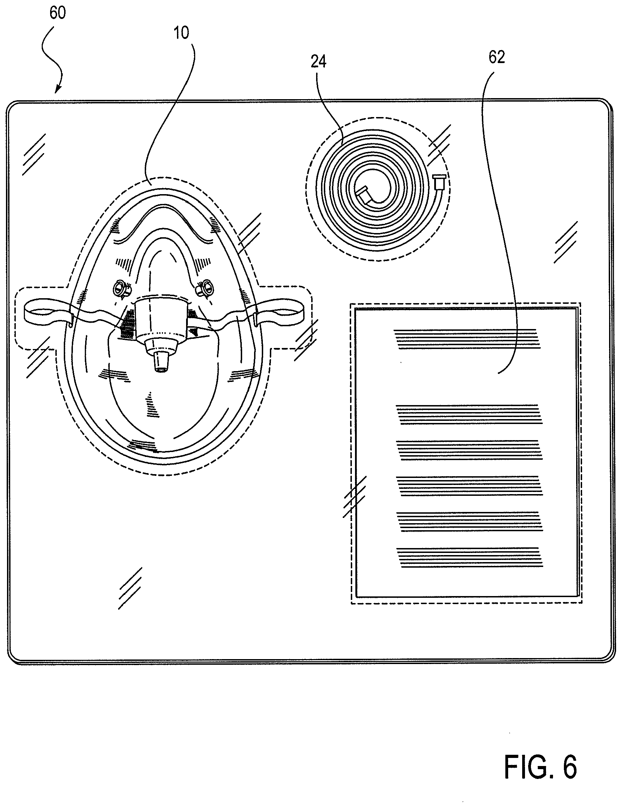

A face mask may be packaged into a kit. A kit may have any component configured to be used with the face mask. A kit may include e.g. a face mask, a sampling conduit, a sensor, an oxygen conduit, a rebreather reservoir, a one way valve, and/or an instruction(s) for use. FIG. 6 shows kit 60 with face mask 10, sampling conduit 24, and instruction for use 62.

A face mask or face mask system or a component used with the face mask may include an alarm, such as alarm 42 shown in FIG. 4. An alarm may provide a signal in response to a result from a component measurement. The alarm may be any (e.g. auditory, vibratory, visual,). An alarm may provide a signal when a level of an expiratory gas is at or different from a threshold amount (e.g. is above or below a threshold amount). In one example, the alarm is auditory and provides a signal when a level of carbon dioxide is different from a threshold level (e.g. when a partial pressure of carbon dioxide is below a threshold level).

Any material can be delivered through the mask to the patient that would benefit the individual. Gas (e.g. room air, oxygen, and/or respired air) may be delivered. Room air, oxygen, and/or respired air may be delivered with or without also delivering an anesthetic agent and with or without a sample being monitored. Room air may be delivered through vents in the mask, through an oxygen line connector, through another connector, or along an unsealed or open edge of the mask. Room air may be mixed with another gas (e.g. oxygen) and delivered.

In one example, oxygen is delivered through an oxygen inlet port. An amount of oxygen delivered may be any therapeutic amount (e.g. 21-100%). The oxygen may be delivered at any flow (e.g. low, medium, or high flow).

Oxygen may be delivered at a relatively low flow rate. In another example, respired air may be delivered with oxygen. A reservoir or bag configured to supply oxygen and respired air may be coupled with the mask. A mask may have a one way valve on one or more exhalation vents to release expired air to the room (e.g. a rebreather or partial rebreather mask) without substantially allowing room air into the mask.

Oxygen may be delivered to the face mask with little or no exhaled air delivered or remaining in the face mask (e.g. the mask or mask system may be a non-rebreather or partial rebreather mask or mask system). An exhalation vent may include a one-way valve configured to allow the release of gas (e.g. exhaled air) from the mask without allowing intake of room air. In one example, oxygen may be delivered using a reservoir bag. A reservoir bag may be connected with a mask using an oxygen line connector or other connector and may be connected with a source of oxygen (e.g. an oxygen tank). A connection between the reservoir bag and the face mask may include a one way valve that prevents inhaled air from entering the reservoir. Any of the components may be connected with a face mask, or may be separate from a face mask. A system including an oxygen face mask of the disclosure may include one or more components for connecting with or using with a face mask.

Oxygen may be delivered at a relatively high flow or pressure (e.g. 4 to 10 L/min) into the face mask (e.g. a Venturi mask). A high flow may in turn cause a percentage of the oxygen in the face mask to be higher or controlled (e.g. more constant).

Alternatively, a device for creating or delivering a nebulized agent (e.g. a nebulizer) or aerosoled agent may be connected with an oxygen line connector or another connector. Any material may be delivered through a nebulizer device. For example, a bronchodilator or glucocorticoid may be delivered. In one example, albuterol is delivered. In another example, ipratropium may be delivered. This may be especially beneficial for a patient suffering from COPD or asthma.

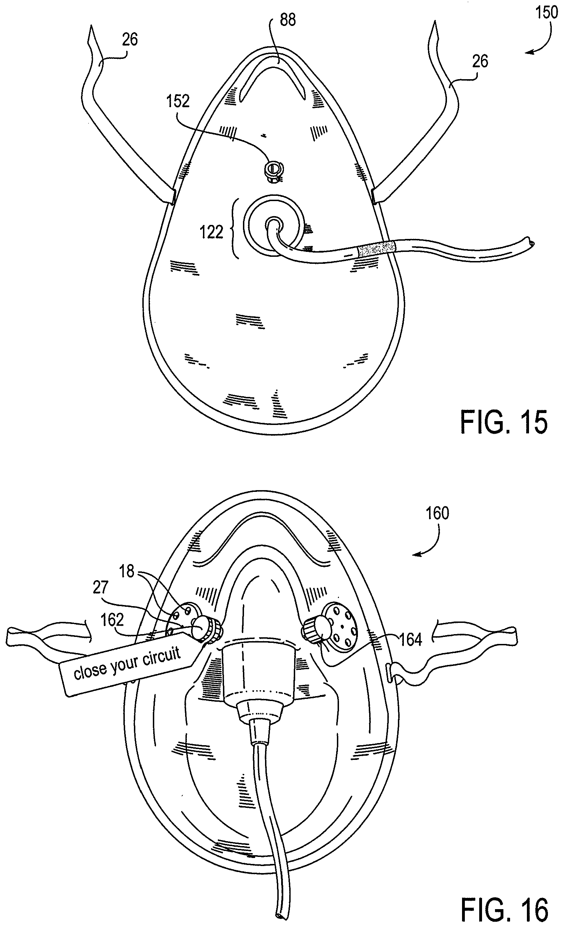

A face mask could instead have a single sampling port 152 located along the midline of the oxygen face mask as shown FIG. 15. A sampling port may be located between the nose and the mouth. A sampling port may be located above and away from a face mask component configured for delivering oxygen. A mask with a low profile sampling port at the midline may be easy to use and minimally obstructive to the patient's view. In one example, the opening of a sampling port may point downwards (e.g. away from the user's eyes).

A face mask may be any shape that fits over a portion of the patient's face to provide oxygen and obtain a gas sample. A face mask may be generally diamond shaped or may be oval. A mask may have features to accommodate contours of the face (e.g. the nose, chin, cheeks). Different masks may have features for different individuals (e.g. large patient, obese patient, pediatric patient). A face mask may be configured to cover the nose and mouth. A mask may cover the nose and part of the mouth. A face mask may cover the nose and all of the mouth. A mask may be configured for use on a mammal (e.g. a human). A face mask may exclude covering the eyes.

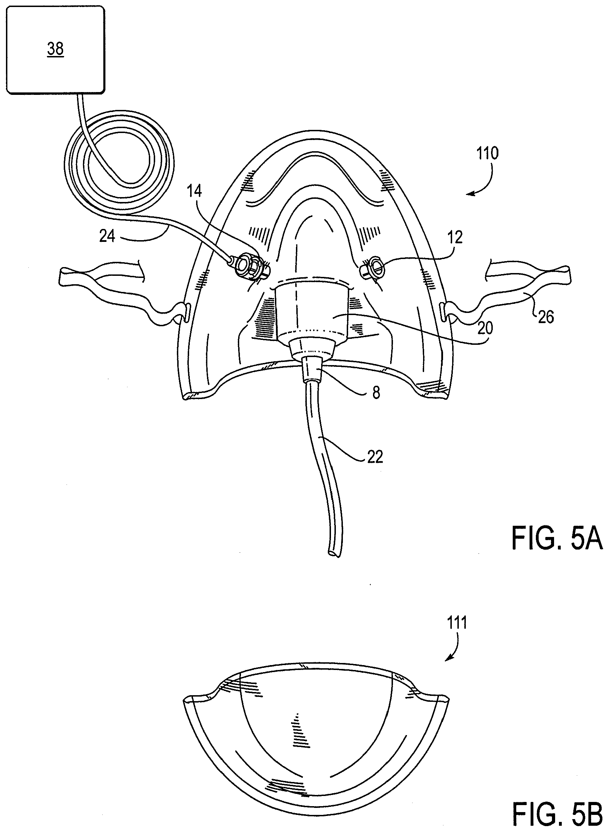

According to one aspect, a mask with lateral sampling ports may be a short mask, having a top portion without a bottom portion. A short mask allows access to the lower part of the patient's face (e.g. a patient's mouth). FIG. 5A shows short face mask 110 with various features, including left and right lateral sampling ports 12, 14, strap(s) 26, and oxygen inlet port 20. Right lateral sampling port 14 is connected with sensor 38 through sampling conduit 24 for sampling an expiratory gas. Left lateral sampling port 12 is not being used in this example. A short mask may be directly manufactured, or may be made by cutting a full (e.g. long mask) to remove a bottom portion of the face mask. FIG. 5B shows a bottom portion 111 of a face mask that has been removed from a top portion, to create a face mask such as short face mask 110 shown in FIG. 5A.

In another aspect, a mask may not have exhalation ports. For example, a mask open at the bottom, such as a short mask shown in FIG. 5 might not need exhalation ports. Masks having a sampling port closer to the bottom of the mask are cumbersome to use in a procedure in which the bottom part of the mask may be removed but expiratory gas(es) still need to be measured. Access to the lower part of the patient's face may be for any reason. A short mask may allow an endotracheal tube, endoscope, or echocardiogram probe to be inserted into the patient's mouth. An endotracheal tube may provide oxygen and anesthesia to the patient. In one example, access to the patient's mouth may allow nourishment or fluids to be provided. In another example, access may allow a procedure to be performed, such as a facial procedure or surgery or dental work.

A mask may be any size to fit an individual. In one example, a mask may be configured to fit onto most average adults. A mask may be configured to fit an especially large or obese individual (e.g. may be larger or may have a different shape). In another example, a mask may be configured to fit a child. In another example, a mask may be configured to fit a baby.

A mask may have a sealing portion to removably seal or connect with the user's face. A sealing portion may retain gas in the mask; a sealing portion may reduce or prevent expiratory gas and/or oxygen from escaping from a mask. A sealing portion may be an edge portion of the mask. A mask may have special features (e.g. silicone edges, a sealing air pocket, lubricant, etc.) to improve the connection or removal of a mask relative to the face or to make a mask more comfortable when in use.

A mask may have any type of fastener or holder to hold the mask in place (e.g. an elastic loop to go behind the head, loops to go around the ears, etc.).

One aspect is a face mask to deliver oxygen to a user, including a superior mask portion having two opposing lateral sides and a bottom side, wherein the superior mask portion is adapted to cover the user's nose and the bottom side is adapted to be superior to the user's mouth when the face mask is in position on the user, and an inferior mask frame portion connected with the superior mask portion and surrounding a generally central open portion, wherein the central open portion is adapted to be over the user's mouth when the face mask is in use on the user, and an oxygen port for delivering oxygen to the user. A face mask with an open portion may provide better access to a user's face, mouth, and/or nose for diagnostic equipment, medical devices, surgical equipment, and/or a caregiver's hands. A face mask with an open portion may provide better visibility to a caregiver for performing a procedure. A face mask with an open portion may be especially useful for performing a procedure on or near the user's face or through the face mask, such as a dental procedure, an esophageal procedure, a facial procedure, and/or an other oral procedure. In a particular example, endoscopy may be performed.

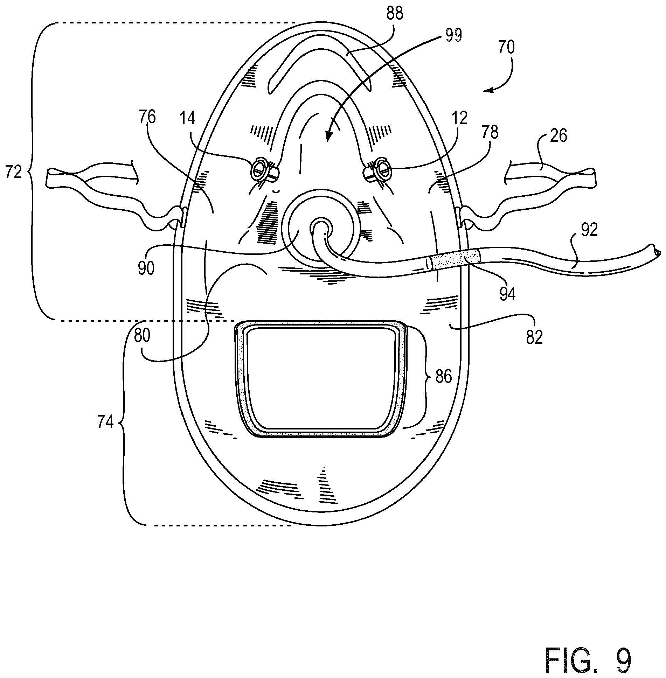

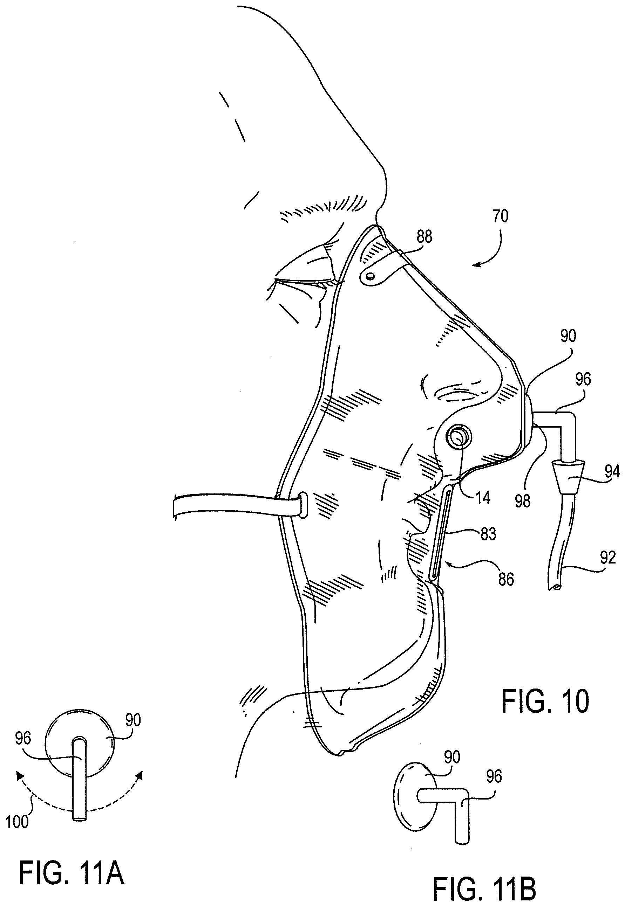

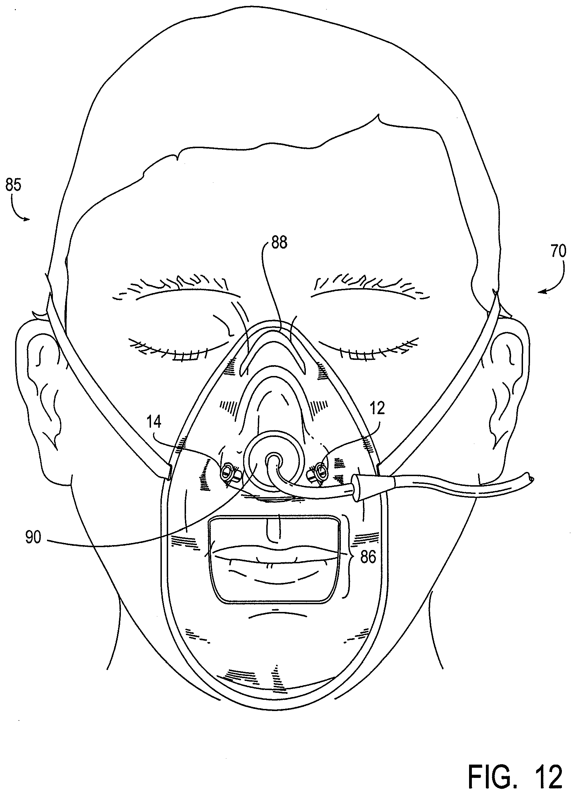

FIGS. 9, 10, and 12 show face masks with a generally central open portion. FIG. 9 shows a front view of a mask with a central open portion, and FIGS. 10 and 12 show front and side views of a mask with a central open portion in position on a user. Mask 70 has a superior mask portion 72 and an inferior mask frame portion 74 with an opening 86. Superior mask portion 72 includes first lateral side 76 and second lateral side 78 on an opposing side of the midline from the first lateral side, and bottom side 80. Superior mask portion 72 is superior to a user's mouth when the face mask is in position on a user. Superior mask portion 72 has reservoir 99, a space that may provide a gas and comfort and ease of breathing. Superior mask portion may be contoured or shaped to contact and/or encompass a user's face when the mask is in use on the user. In a particular example, the opposing lateral sides are contoured or shaped to contact and/or encompass a user's face when the mask is in use on the user.

Inferior mask frame portion 74 may also be contoured or shaped to contact and/or encompass a portion of a user's face when the mask is in use on the user. Inferior mask frame portion may be configured to allow a user to open his mouth while the mask is in position on the user and while keeping the superior mask portion in position (e.g. the inferior mask frame portion may accommodate movement of the jaw of the user without moving the superior mask portion out of position). In some aspects, an inferior mask frame portion may provide additional space near the user's chin to allow movement.

Inferior mask frame portion 74 is inferior to the superior mask portion and forms frame 82 around the bottom and lateral sides of the opening. Although the opening is shown as a generally rectangular shape in these figures, the opening can be any shape as long as it allows access to an area larger than the mouth, so that it can be a circular, an elliptical, a hexagonal, an oval, a rounded rectangular, a rounded square, a square or another shape, and the frame can be any corresponding shape to encompass bottom and lateral portions of the opening. An opening may be generally symmetrical about the midline of the face mask (e.g. in a generally central location), but could instead be off-center or irregularly shaped. The opening may be in any location inferior to the superior mask portion.

An opening in a face mask may be any size that can accommodate a surgical, diagnostic, or other device and/or a caregiver's hands. In some aspects, an opening is larger than a user's mouth (e.g. a user's open mouth). In some other aspects, an opening is larger than an endoscope. In some other aspects, an opening is larger than an echocardiogram probe. There may be different sizes of face masks and/or different size openings for different users. A face mask may be sized to fit a face of an infant, a child, or an adult and an opening may be sized accordingly. A face mask and/or its associated opening may be sized to accommodate a particularly large person or an obese person. An opening may be larger than a user's mouth. An opening may be larger than a user's open mouth when a scope or other device is inserted into the user's mouth (or nose). An opening may be larger than a scope or other device when a scope or other device is inserted into the user's mouth or nose. An opening may be sized to not contact a scope when a scope is in place in a user's mouth (or nose). An opening may be sufficiently large to allow a surgical or diagnostic procedure to be performed on part of a user's face. An opening may be sufficiently large to allow a physician's hands or other caregiver hand's to manipulate a scope or other devices into a user's mouth. An opening may be larger than about 4 cm² , larger than about 5 cm², or larger than about 6 cm². An opening may be smaller than about 6 cm². An opening that is in the shape of a circle (or a square) may have a diameter (or a side) larger than 2 cm, larger than about 3 cm, larger than about 4 cm, larger than about 5 cm, or larger than about 6 cm. A diameter of a circle or a side of a square may be smaller than about 6 cm.

Another aspect of the disclosure provides method of using a face mask, the face mask comprising a superior mask portion having two opposing lateral sides and a bottom side, wherein the superior mask portion is adapted to cover the user's nose and the bottom side is adapted to be superior to the user's mouth when the face mask is in position on the user, and an inferior mask frame portion connected with the superior mask portion, comprising a mask frame around a generally central open portion, wherein the generally central open portion is adapted to be over the user's mouth when the face mask is in use on the user and has an initial size and an initial shape, the method including the steps of positioning the face mask on a user, and inserting a device through the generally central open portion while maintaining the initial size and the initial shape of the generally central open portion.

A mask with a generally central opening may be made of any biocompatible material (or materials) suitable for placing on a user. Superior mask portion and inferior mask frame portion of the face mask may be made of the same materials or may be made of different materials.

An inferior mask frame portion contacting a generally central opening may be configured to hold a shape (e.g. to remain open or hold an initial shape). It may be configured to maintain a size (e.g. to hold an initial size). It may be configured to hold a shape and/or hold a size in the absence of any applied force (such as an applied opposing force from a scope or other device). An inferior frame portion contacting a generally central opening may be inflexible, non-compliant, rigid, and/or stiff. FIG. 10 shows a face mask with rim 83 around generally central opening 86. A rim may go all the way around or partway around a generally central opening. In some embodiments, a rim may be inflexible, non-compliant, resilient, rigid, and/or stiff. In some embodiments, a rim may be compliant and/or flexible and may be configured to form a seal with a face of a user (e.g. be a gasket).

Having an opening in a face mask may allow a face mask, or part of a face mask (e.g. an inferior mask frame portion), to bend, pull up (e.g. pull superiorly), twist or otherwise move, especially in response to handling or face movement. Portions of the inferior mask frame portion may include a material with a stiffness greater than a stiffness of a portion of the superior mask portion. In particular, the inferior mask frame portion (or a portion thereof) may include a reinforced material that is a reinforced version of a material used elsewhere in the face mask, such as in the superior mask portion. A reinforced material may be reinforced in any way and using any material that produces a stronger or more resilient material that is safe for use on a user, such as using a thicker material and/or including a fabric and/or a plurality of fibers, particles, and/or threads. A reinforcement material may be, for example, a cloth, metal, or a polymer. A stiff material may extend to the generally central opening, and may generally surround the opening. A stiff material may extend throughout the inferior mask frame portion.

A face mask may have other features to help hold the face mask in place on a user. FIG. 9 shows strap(s) 26 coupled with first lateral side 76 and second lateral side 78 and configured to wrap behind a user's head and hold a face mask in place when in use on user 85. A face mask may have one or more straps. Two straps may meet and join, or two or more straps may be generally parallel or crisscross. A face mask to hold a face mask to a user's face may have an adhesive material partway or all around an outside edge of a face mask and/or may have an adhesive material partway or all around the opening or around another part of the face mask. An adhesive material may removably attach a part of a face mask to a user's face. In a particular example, an adhesive material on a face mask is configured to removably attach an outer portion of the mask to a user. A face mask may include a material (e.g. a foam; shape-memory foam) that can conform to the user's face and may help hold the mask in place, or otherwise provide support or comfort. FIGS. 9, 10, and 12 show shapeable bridge 88 configured to conform to a portion of a user's face and help hold a face mask in place.

A face mask with a generally central opening may have none, one, two, or more than two sampling ports. FIGS. 9 and 12 show a face mask with left lateral sampling port 12 and right lateral sampling port14 on opposing sides of a midline. A port may be located anywhere on the mask and may have any of the characteristic(s) as described elsewhere in the disclosure.

A face mask with a generally central opening may have a gas port extending through the face mask. FIGS. 9, 10, and 12 show gas port 90 in the superior mask portion. FIGS. 11A-B show different views of a gas port and elbow. A gas port may have an internal end in a mask reservoir and may be partially, totally, or not at all directly above the nares when in place on a user. A gas port may be superior to, inferior to, or at the same level as the nares when the face mask is in place on a user. FIGS. 9-12 show different views of gas port 90 configured to receive a gas (e.g. oxygen) and to deliver the gas (oxygen) through or to a face mask. Oxygen is delivered through an oxygen conduit, through tubing 92, through elbow connector 94, elbow 96, channel 98, and through gas port 90. Elbow 96 is formed of a material stiffer than another portion of the gas conduit (such as tubing). An elbow may be made of a sufficiently stiff material to be held a bent position while in use. An elbow may be sufficiently stiff to not change its shape during use, or it may be configured to be bent from a first shape into a second shape and maintain the bent configuration while in use. In some embodiments, an elbow may define an angle between about 80 degrees and about 180 degrees, between about 90 and 180 degrees, or between about 90 and 135 degrees. In some particular embodiments, an elbow may define an angle of about 90 degrees. In some embodiments, all or part of a stiff ("elbow") portion may be located within about 2 inches, within about 1.5 inches, within about 1 inch, or within about 0.5 inches from the port.

Elbow 96 is further configured to move (rotate or swivel), along with another part(s) of the conduit, and may be configured to move while the face mask is in place on a user and when the elbow connected with the face mask. Elbow 96 may be rotated in order to move tubing 92. As indicated by arrow 100, an elbow (and part of the conduit) may rotate (swivel) any amount. In some embodiments, an elbow may rotate up to (and including) 90 degrees, up to (and including) 180 degrees, or up to (and including) 360 degrees. Elbow and tubing may be moved for any reason, such as to prevent them from interfering with a procedure that is performed through the opening or a procedure being performed close to the mask, or to increase the comfort of the user, to increase the convenience of a caregiver, or for any another reason.

One aspect of the disclosure is a face mask to deliver oxygen to a user, including a superior mask portion having two opposing lateral sides and a bottom side, wherein the superior mask portion is adapted to cover the user's nose and the bottom side is adapted to be superior to the user's mouth when the face mask is in position on the user, and an inferior mask portion connected with the superior mask portion and surrounding a generally central membrane wherein the membrane is adapted to be over the user's mouth when the face mask is in use on the user, and an oxygen port for delivering oxygen to the user. A generally central membrane may be configured to allow a device (e.g. a scope) and/or fingers to move from outside a face mask to inside a face mask (e.g. it may have a perforation). In some aspects, it may be configured to form a seal with a device or scope to thereby reduce or prevent a gas from moving from inside to outside a face mask. In some aspects, a membrane may be removable from a face mask. In a method of using a membrane, a membrane may be placed around an object, at least a portion of an object placed through an opening in a face mask, and a membrane may be connected with the face mask.

Another aspect of the invention includes a face mask to deliver oxygen to a user, the face mask including a connector for connecting a gas conduit with a port on the face mask, wherein the connector is configured to move with at least two degrees of freedom relative to a point on the port. The face mask may be connected to an anesthesia machine, a nebulizer, and/or may be further configured to deliver other agents, including, but not limited to an aerosol, an anesthesia agent, and/or a nebulized agent to the user. A face mask with a connector configured to move with at least two degrees of freedom may be used for any purpose, but may be especially useful for performing a surgical or diagnostic procedure on an upper portion of a user, in particular superior to the T5 dermatome of the user. The face mask may include additional features that are useful in performing such a surgical or diagnostic procedure.

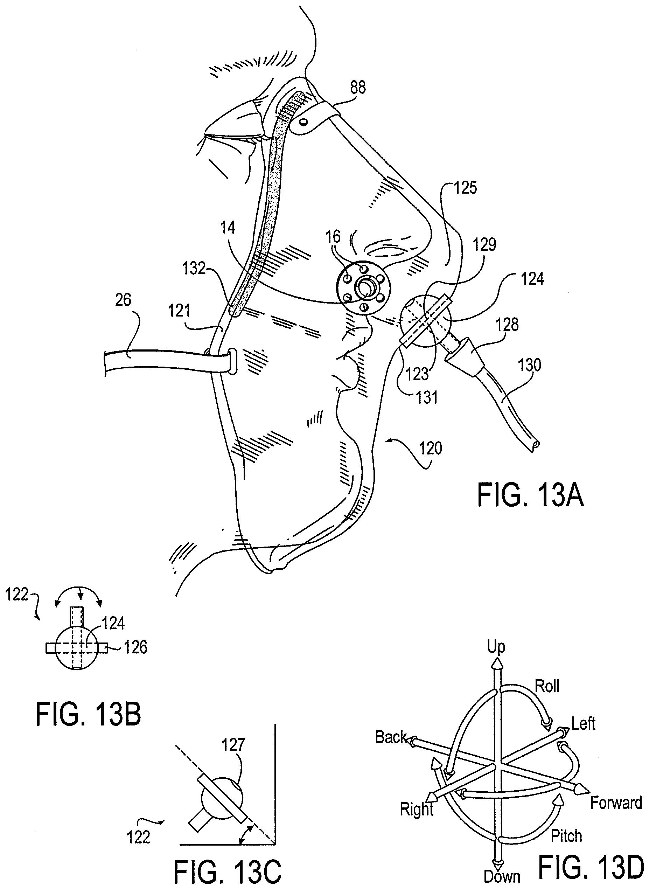

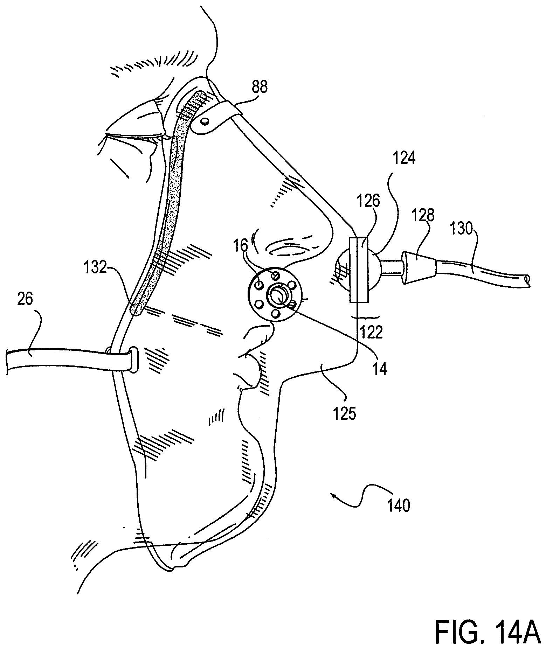

FIGS. 13 A-C and 14A-C show embodiments of face masks and movable gas inlets, including reservoir 125 and joint 122 in different configurations. The joint includes first mating piece 124 and second mating piece 126 which are configured to mate with one another, such as in a ball-in-socket mechanism. The first mating piece, or connector, is configured to move with at least two degrees of freedom. The first mating piece, or connector, may be configured to move with at least two degrees of freedom relative to a point 129 on gas port 123. It may connect with the second mating piece and with a gas conduit. Gas conduit includes joining member 128 and tubing 130. A joining member and/or tubing may be configured to move with at least two degrees of freedom. In some embodiments, a joining member and/or tubing may be configured to move with at least two degrees of freedom relative to a point on the gas port. In some embodiments, gas port 123 is continuous with reservoir 125 so that a gas may be moved through the port and into the reservoir. A joining member may include a region of stiffness that is stiffer than another portion of the gas conduit. A joining member may hold a tubing in a position. A joining member may include an elbow as described above. An elbow may be formed of a material stiffer than another portion of the gas conduit, such as tubing. An elbow may be made of a sufficiently stiff material to hold a bent shape while in use. It may be sufficiently stiff to not change its shape during use, or it may sufficiently flexible or resilient be able to be changed into a shape, and then maintain that shape while in use. In some embodiments, it may be in or made into an elbow defining an angle between about 80 degrees and about 150 degrees. In some particular embodiments, it may be at or made into an elbow defining an angle of about 90 degrees. In some embodiments, all or part of a stiff ("elbow") portion may be within about 2 inches, within about 1.5 inches, within about 1 inch, or within about 0.5 inches from the port.

A connector (and tubing) configured to move with multiple degrees of freedom allows the connector and tubing to be conveniently moved out of the way of a surgical procedure or moved to a more convenient location or moved to a more accessible location. A face mask with a movable connector and/or gas conduit may be useful for any type of surgery, but may be especially useful for surgery in a superior part of a body, especially above the level of the fifth thoracic dermatome (e.g. approximately the nipple line), in which the position of the patient and/or the location of a procedure on the body may interfere with connection or the location of the gas (oxygen) conduit, or may create safety concerns. A movable connector allows the gas conduit to be moved out of the way body habitus of a patient such as a prone or obese patient. A connector may be moved to a position without conduit attached to it, or a connector and tubing may be moved together. In some embodiments, a connector and/or tubing may be configured to move with one, two, three, four, five, or six degrees of freedom. A connector and/or tubing may be configured to rotate with one, two, or three degrees of freedom (see FIG. 13D). A first or second mating piece (or both pieces) may be shaped (e.g. such as being oval shaped) so as to limit the degrees of freedom. The first mating piece may have or may be acted upon by a hold mechanism in order to prevent (further) movement, such as after being placed in a preferred location. A hold mechanism may be any mechanism that prevents or reduces movement, such as a tab that locks the first mating piece in place or a clip that holds the gas conduit in position. In another embodiment, a first mating piece may move relative to a point on a gas port independent of any rotational movement. In some other embodiments, a first mating piece may be positioned close to, but not in line with an edge of a face mask. It may be connected with the port through a short conduit (e.g. tubing or pipe).

A face mask, joint, and conduit may be joined or formed together in any way. In one embodiment, a connector (first mating piece) may be connected with a second mating piece on a face mask (e.g. forming a joint) and then a conduit attached with the connector (first mating piece). In another embodiment, a conduit may be connected with a connector (first mating piece), and then the connector (first mating piece) attached with a second mating piece on face mask to form a joint. In another embodiment, a first mating piece may first be connected with a second mating piece and with a gas conduit, and then connected with a port on a face mask. As can be seen from FIGS. 13 A,B, and C first mating piece 124 has channel 127 to allow a gas to pass therethrough, such as from a conduit to a face mask and into reservoir 125. Different orientations of the reservoir and joint are shown in FIGS. 13A and FIGS. 14A-C. As shown in FIG. 13A, the second mating piece attaches to an inferior surface of the mask at reservoir 125. An inferior surface of the mask defines an angle with a transverse plane of a user. In can be seen from FIG. 13A and FIG. 13C, the inferior surface and a transverse plane of a user when the mask is in place on a user form an angle. An inferior surface may define an angle from 0 degrees to about 90 degrees (e.g. an acute angle) relative to a frontal plane of a user when a mask is on a user.

The first and second mating pieces may fit together in any way that allows movement with at least two degrees of freedom. A first mating piece 124 may have a rounded portion (e.g. a ball or ball-shaped end) that articulates with a collar-shaped second mating piece 126, as shown in FIGS. 13 B, C. A second mating piece, such as a collar, may be in any position that allows gas flow and ease of face mask use. A second mating piece may define an angle from about 0 degree angle to about a 90 degree angle relative to a transverse plan of a user when the mask is on the user, as shown in FIGS. 14A-C. In one particular example, the second mating piece defines about a 45 degree angle with a transverse plane of a user when the mask is on the user.

A face mask with a movable connector and/or movable gas conduit may include any of the features or characteristics described elsewhere. In some embodiments, it may be configured to cover a user's nose and at least partially cover a user's mouth. In other embodiments, it may include at least one sampling port, at least one lateral sampling port, at least two sampling ports, or at least two lateral sampling ports on opposite sides of a midline of the face mask.

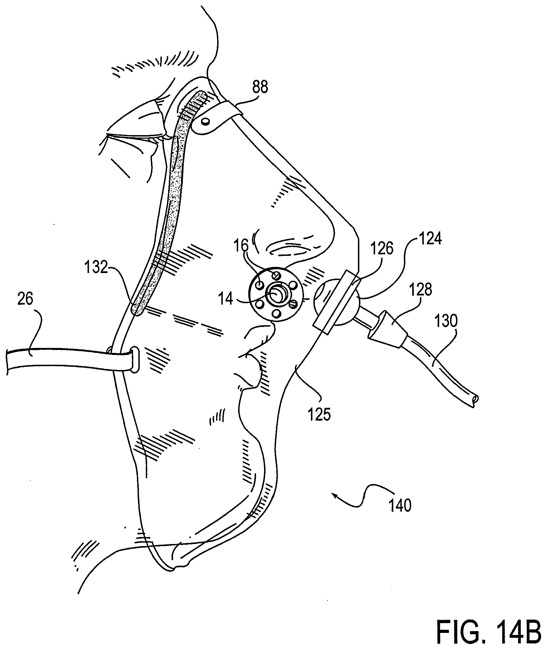

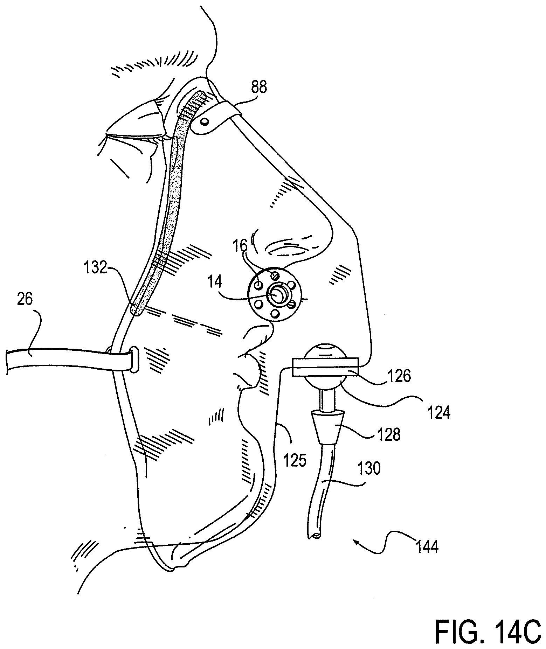

FIGS. 13A and 14 show a removable, biocompatible adhesive material 132 along an edge of face mask 120 and face mask 140 for holding a face mask to a user's face. An adhesive material may be used in addition to, or instead of a head strap. An adhesive material may be especially useful when performing a diagnostic, surgical, or other procedure in which a head strap interferes or is otherwise difficult to use.

A face mask with a movable connector may be made with an inexpensive, comfortable, malleable, non-reactive material, such as silicone. However, a face mask may instead be made from another material(s), and these materials may be more expensive, less comfortable, less malleable, and/or may have other drawbacks. Use of such another material may however be beneficial, such as while performing a diagnostic, surgical or other procedure on a user above about the T5 dermatome level of the user, in order to reduce or prevent patient injury. Procedures performed with surgical or other equipment in close proximity to a source of oxygen, which is highly flammable, have a higher risk of causing a fire. Fires, started by a spark or heat from a piece of equipment igniting oxygen gas, can melt face masks on patients, causing patient injury and scarring, as well as creating dangerous situations physicians, and other caregivers. In some embodiments, a face mask may be made from a heat resistant material, flame resistant material, or fireproof material such as a heat or flame resistant or fireproof polyvinyl fluoride or polyvinyl chloride or other heat resistant material. In some embodiments Teknor APEX® 3800 and/or Teknor DEHP free APEX 3801 (60, 65, 70, 75, 80, 85, or 90 shore (Shore A, 15 sec)) may be used.

A face mask, and in particular, a facemask with a movable connector may have additional features that may be useful while performing a surgical or other procedure or may encourage safer practices. A face mask or tubing for supplying oxygen (or another gas) may be partially or entirely a warning color, such as red, orange, or yellow, or bright yellow-green, to provide warning that a higher-than-normal risk for fire is present. A mask may further have other visual cues, such as a downwardly pointing arrow, to warn a physician(s) and other caregiver(s) to reduce the flow of oxygen to reduce risk of fire. A sign may be placed on or near a tubing for oxygen use, e.g. at the distal end of the oxygen inflow tubing near the oxygen inflow source, with a warning, such as "Use Low Flows!"

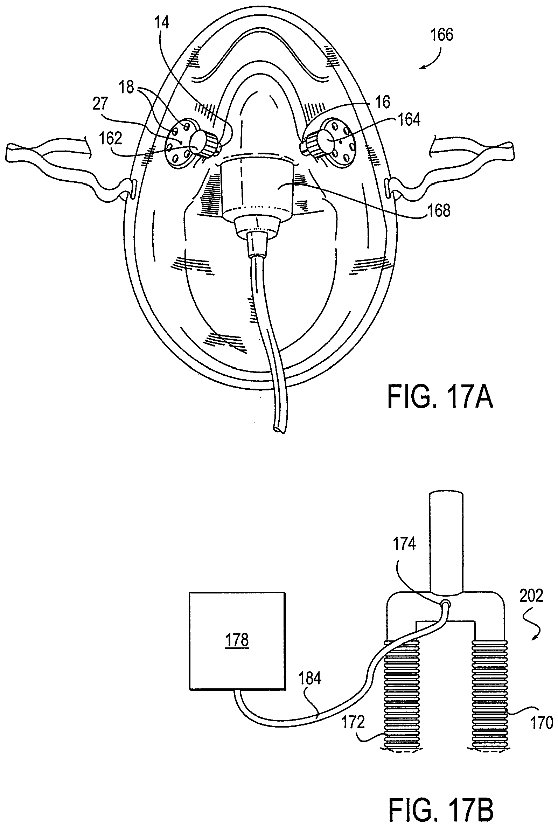

Another aspect of the disclosure includes a method of using a face mask and an anesthesia breathing circuit, the face mask including at least one sampling port to sample an expiratory gas from a user and the anesthesia breathing circuit configured to provide an anesthetic agent and positive pressure ventilation to the user, the method including the steps of: removing a cap from a sampling port on the face mask, removing a sampling conduit from a sensor port on the anesthesia breathing circuit to thereby expose an opening on the sensor port, coupling the cap to the sensor port to thereby close the opening on the sensor port, and coupling the sampling conduit to the sampling port on the face mask. FIGS. 17 A-B show devices, systems, and methods that may be used, for example, to provide oxygen and to provide an anesthesia agent, to remove carbon dioxide and to remove an anesthesia agent, and to monitor a gas(es) (e.g. an exhaled gas) for a user undergoing a diagnostic, exploratory, surgical or other procedure. The devices, systems and methods may be used, with a face mask, a gas sensor, and an anesthesia breathing circuit and methods of using the devices and systems. The devices, systems, and methods provide for monitoring a gas from an anesthesia breathing circuit and/or an expiratory gas.

FIG. 17B shows sensor port 174 on anesthesia breathing circuit 202 to use for assaying a breathing circuit gas. As shown in FIG. 17B, a first end of sampling conduit 184 has been connected with gas sensor 178 of an anesthesia apparatus and a second end of sampling conduit 184 has been connected with sensor port 174 on anesthesia breathing circuit 202, allowing a breathing circuit gas to flow to gas sensor 178 and be analyzed.

FIG. 17A shows face mask 166 with left cap 164 on left lateral sampling port 16 and right cap 162 on right lateral sampling port 14. Although both ports are shown with a cap, a face mask could have a left lateral sampling port covered with a cap, a right lateral sampling port covered with a cap, or both lateral sampling ports covered with caps. If there are more than two sampling ports, each sampling port may have a cap. The caps are removable from the face mask. The caps may be identical to one another or may be different from one another. A cap may cover or be connected with a sampling port in any way (e.g. they may be fitted together, screwed together, connected via a luer lock connection). A cap may be near a port, but not connected with it. For example, a cap may be placed separately from a port or separately from a face mask in a face mask kit, such as a kit shown in FIG. 6, or a removable cap may be connected (such as by a removable adhesive material) to another portion of a face mask. A cap may labeled, such as with a warning ("Close Your Circuit!"). A face mask with one or more removable caps may be placed on a user before a cap is removed.

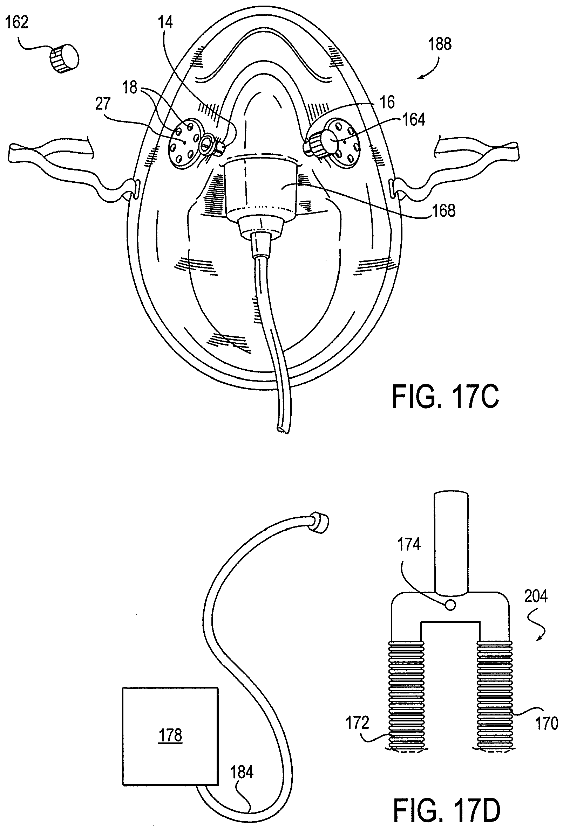

FIG. 17D shows sampling conduit 184 being removed from the anesthesia breathing circuit, exposing sensor port 174. Anesthesia breathing circuit 204 is "open" and (temporarily) unable to provide positive pressure ventilation.

FIG. 17C shows right cap 162 being removed from a right lateral sampling port on face mask 188, exposing right lateral sampling port 14. Left cap 164 remains in place.

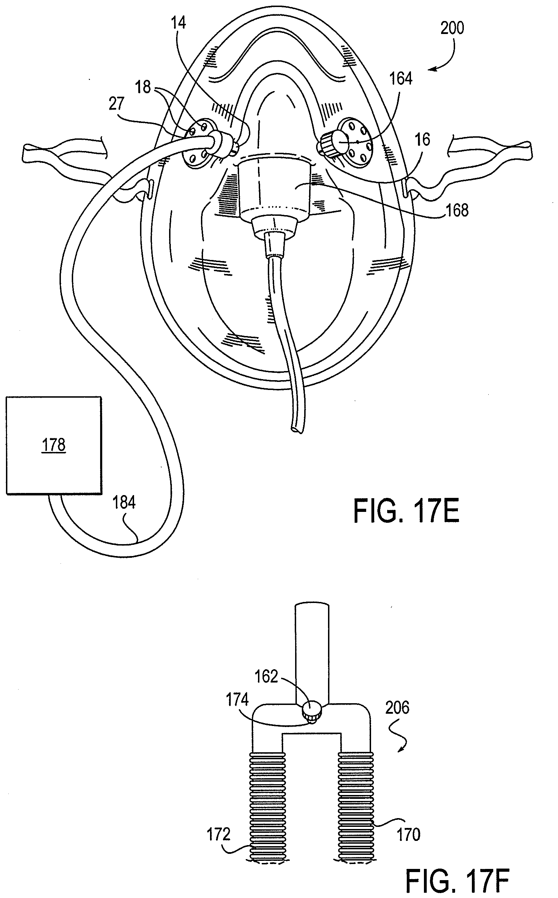

FIG. 17F shows right cap 162 coupled with anesthesia breathing circuit sensor port 174, closing the sensor port, and allowing (the now closed) anesthesia breathing circuit 206 to provide positive pressure ventilation.

FIG. 17E shows sampling conduit 184 coupled with right lateral sampling port 14 on face mask 200, allowing gas from inside face mask 200 to flow through sampling conduit 184 to gas sensor 178, where a level of a gas may be sensed, analyzed, communicated, and/or displayed.

Any characteristic of a gas (e.g. a breathing circuit gas or an expiratory gas) may be analyzed by a gas sensor and a gas sensor may have any format or composition for assaying (e.g. chemical, light, other energy) as long as it can sense a gas. It may further analyze a component(s) of a gas and/or provide an indication of a level or amount of a gas (e.g. an audible or visual display). It may be connected with an alarm configured to provide a signal, for example if a threshold level of a gas is different from a desired amount of gas. In one example, carbon dioxide may be analyzed.

A face mask according to the disclosure and/or a method of using a face mask may have or be combined with any of the other characteristics, features, and/or methods described herein.

As for additional details pertinent to the present invention, materials and manufacturing techniques may be employed as within the level of those with skill in the relevant art. The same may hold true with respect to method-based aspects of the invention in terms of additional acts commonly or logically employed. Likewise, reference to a singular item, includes the possibility that there are plural of the same items present. More specifically, as used herein and in the appended claims, the singular forms "a," "and," "said," and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Unless defined otherwise herein, all technical and scientific terms used herein have the same meaning as commonly understood by one of ordinary skill in the art to which this invention belongs. The breadth of the present invention is not to be limited by the subject specification, but rather only by the plain meaning of the claim terms employed.

## Claims

1. A face mask to cover a user's nose and at least partially cover a user's mouth and configured to deliver oxygen to a user and provide fluid communication with an external conduit, the face mask comprising:
a reservoir (19) for containing a pocket of gas;
an oxygen inlet port (20) for delivering oxygen to the reservoir;
first and second lateral sampling ports (12, 14) on opposing sides of a midline of the face mask, the two lateral sampling ports located above the oxygen inlet port and configured to sample gas from the reservoir;
first and second vents (16, 18) on opposing sides of the midline of the face mask and configured to release gas from inside to outside the mask, the first lateral sampling port located near the first vent to allow sampling of exhaled gas as gas is moved past the first lateral sampling port, the second lateral sampling port located near the second vent to allow sampling of exhaled gas as gas is moved past the second lateral sampling port; and
a mating part on each of the first and second lateral sampling ports that is configured to enable connection of the respective lateral sampling port to the external conduit.

2. The face mask of claim 1 wherein a center of a sampling port is at least about 20 mm away from a center of the oxygen inlet port.

3. The face mask of claim 1 wherein the first and second sampling ports are at a distance of 1 mm to 15 mm from the respective first and second vents.

## Patentansprüche

1. Gesichtsmaske zum Bedecken der Nase eines Benutzers und zum wenigstens teilweisen Bedecken des Mundes eines Benutzers, und konfiguriert zum Zuführen von Sauerstoff zu einem Benutzer und zum Bereitstellen von Fluidkommunikation mit einer externen Leitung, wobei die Gesichtsmaske Folgendes umfasst:
ein Reservoir (19) zum Aufnehmen eines Gasvorrats;
einen Sauerstoffeinlassanschluss (20) zum Zuführen von Sauerstoff zu dem Reservoir;
einen ersten und zweiten seitlichen Probenahmeanschluss (12, 14) auf gegenüberliegenden Seiten einer Mittellinie der Gesichtsmaske, wobei sich die zwei seitlichen Probenahmeanschlüsse über dem Sauerstoffeinlassanschluss befinden und zum Nehmen von Gasproben aus dem Reservoir konfiguriert sind;
eine erste und eine zweite Lüftung (16, 18) auf gegenüberliegenden Seiten der Mittellinie der Gesichtsmaske, konfiguriert zum Freisetzen von Gas vom Innern zum Äußern der Maske, wobei sich der erste seitliche Probenahmeanschluss in der Nähe der ersten Lüftung befindet, um das Nehmen von Proben von ausgeatmetem Gas zuzulassen, während sich Gas am ersten seitlichen Probenahmeanschluss vorbei bewegt, wobei sich der zweite seitliche Probenahmeanschluss in der Nähe der zweiten Lüftung befindet, um ein Nehmen von Proben von ausgeatmetem Gas zuzulassen, während sich das Gas am zweiten seitlichen Probenahmeanschluss vorbei bewegt; und
ein Gegenstück an jedem der ersten und zweiten seitlichen Probenahmeanschlüsse, das zum Ermöglichen einer Verbindung des jeweiligen seitlichen Probenahmeanschlusses mit der externen Leitung konfiguriert ist.

2. Gesichtsmaske nach Anspruch 1, wobei eine Mitte eines Probenahmeanschlusses einen Abstand von wenigstens etwa 20 mm von einer Mitte des Sauerstoffeinlassanschlusses hat.

3. Gesichtsmaske nach Anspruch 1, wobei sich der erste und zweite Probenahmeanschluss in einem Abstand von 1 mm bis 15 mm von der jeweiligen ersten und zweiten Lüftung befinden.

## Revendications

1. Masque facial destiné à couvrir le nez d'un utilisateur et à couvrir au moins partiellement la bouche d'un utilisateur et configuré pour délivrer de l'oxygène à un utilisateur et assurer une communication fluidique avec un conduit externe, le masque facial comprenant :
un réservoir (19) destiné à contenir une poche de gaz ;
un orifice d'admission d'oxygène (20) destiné à délivrer de l'oxygène au réservoir ;
des premier et second orifices d'échantillonnage latéraux (12, 14) sur des côtés opposés d'une ligne centrale du masque facial, les deux orifices d'échantillonnage latéraux étant situés au-dessus de l'orifice d'admission d'oxygène et étant configurés pour échantillonner le gaz provenant du réservoir ;
des premier et second évents (16, 18) sur des côtés opposés de la ligne centrale du masque facial et configurés pour évacuer le gaz depuis l'intérieur vers l'extérieur du masque, le premier orifice d'échantillonnage latéral étant situé près du premier évent pour permettre l'échantillonnage du gaz exhalé lorsque le gaz passe devant le premier orifice d'échantillonnage latéral, le second orifice d'échantillonnage latéral étant situé près du second évent pour permettre l'échantillonnage du gaz exhalé quand le gaz passe devant le second orifice d'échantillonnage latéral ; et
une pièce d'accouplement sur chacun des premier et second orifices d'échantillonnage latéraux qui est configurée pour permettre la connexion de l'orifice d'échantillonnage latéral respectif au conduit externe.

2. Masque facial selon la revendication 1 dans lequel un centre d'un orifice d'échantillonnage est écarté d'au moins environ 20 mm d'un centre de l'orifice d'admission d'oxygène.

3. Masque facial selon la revendication 1 dans lequel les premier et second orifices d'échantillonnage se trouvent à une distance de 1 mm à 15 mm des premier et second évents respectifs.
